# EUROPÄISCHE PATENTSCHRIFT

(11) **EP 0 548 618 B1**
(45) Veröffentlichungstag und Bekanntmachung des Hinweises auf die Patenterteilung: **27.09.1995**
(21) Anmeldenummer: 92120623.1
(22) Anmeldetag: 03.12.1992
(51) Int. Cl.: C07C 221/00, C07C 225/22

(54) **Verfahren zur Herstellung von 2-Aminobenzaldehyden**
Process for the preparation of 2-aminobenzaldehydes
Procédé de préparation de 2-amino-aldéhyde benzoique

(30) Priorität: 20.12.1991 DE 4142173
(43) Veröffentlichungstag der Anmeldung: 30.06.1993
(73) Patentinhaber: BASF Aktiengesellschaft, 67063 Ludwigshafen (DE)
(72) Erfinder: Hagen, Helmut, Dr., W-6710 Frankenthal (DE); Dupuis, Jacques, Dr., W-6700 Ludwigshafen (DE); Roetsch, Thomas, Dr., W-6700 Ludwigshafen (DE)

(56) Entgegenhaltungen:
- EP-A- 0 144 690

## Beschreibung

Die vorliegende Erfindung betrifft ein neues Verfahren zur Herstellung von 2-Aminobenzaldehyden durch Behandlung von Dibenzotriazabicyclononanen mit Säure.

2-Aminobenzaldehyde sind wertvolle Zwischenprodukte zur Herstellung von Farbstoffen oder Wirkstoffen (s. z.B. Tetrahedron, Band 36, S. 2359 bis 2408, 1980).

Aus Inorganic Chemistry, Band 16, Seiten 1721 bis 1725, 1977, ist nun bekannt, daß 2-Aminobenzaldehyd in Lösung oder sogar in festem Aggregatzustand schon bei Raumtemperatur einer Selbstkondensation unterliegt, weshalb eine längerfristige Lagerung dieser Verbindung bei Raumtemperatur unrentabel ist, da die Kondensationsprodukte entweder überhaupt nicht oder nur sehr schwer wieder in den freien 2-Aminobenzaldehyd übergeführt werden können.

Weiterhin beschreibt die EP-A-144 690 die Herstellung von Dibenzotriazabicyclononanen. Diese Verbindungen, die dort als verkappte 2-Aminobenzaldehyde bezeichnet werden, zeigen bei Umsetzungen im wesentlichen die Reaktion der entsprechenden 2-Aminobenzaldehyde.

Trotz dieser Umsetzungsmöglichkeiten kann es in vielen Fällen von Vorteil sein, nicht die Dibenzotriazabicyclononane, sondern die jeweiligen freien 2-Aminobenzaldehyde selbst zu verwenden. Dies trifft insbesondere dann zu, wenn die Aminoverbindungen, die bei der Umsetzung der Dibenzotriazabicyclononane als Reaktionsprodukte entstehen, zu unerwünschten Nebenreaktionen oder Störungen führen können.

Aufgabe der vorliegenden Erfindung war es daher, ein neues Verfahren zur Herstellung von 2-Aminobenzaldehyden bereitzustellen, das von den entsprechenden Dibenzotriazabicyclononanen ausgeht und mittels dessen die Zielprodukte auf einfache Weise und hoher Ausbeute und Reinheit erhalten werden können.

Es wurde nun gefunden, daß die Herstellung von 2-Aminobenzaldehyden der Formel I 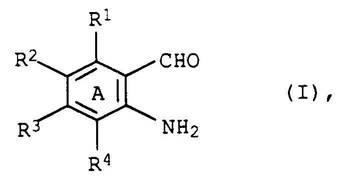
in der R¹, R², R³ und R⁴ gleich oder verschieden sind und unabhängig voneinander jeweils Wasserstoff, Halogen, C₁-C₄-Alkyl, C₃-C₇-Cycloalkyl oder Phenyl bedeuten und der Ring A benzoanelliert sein kann, durch Umsetzung von Triazanonanen der Formel II 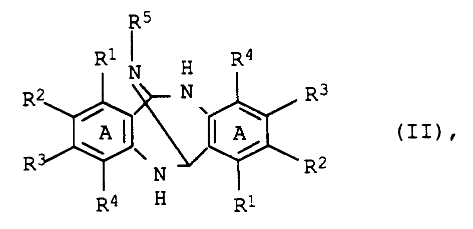
in der R¹, R², R³, R⁴ und der Ring A jeweils die obengenannte Bedeutung besitzen und R⁵ Wasserstoff, gegebenenfalls substituiertes C₁-C₁₀-Alkyl oder C₃-C₇-Cycloalkyl bedeutet, mit Säure vorteilhaft gelingt, wenn man ein Triazanonan der Formel II bei einer Temperatur von 10 bis 120°C in Gegenwart von Wasser mit einer Säure behandelt und den resultierenden 2-Aminobenzaldehyd der Formel I mittels Wasserdampfdestillation abtrennt.

Alle in den obengenannten Formeln I und II auftretenden Alkylgruppen können sowohl geradkettig als auch verzweigt sein.

Wenn in der obengenannten Formel II substituierte Alkylgruppen auftreten, so können als Substituenten z.B. Halogen, wie Fluor, Chlor oder Brom, Hydroxy, C₁-C₄-Alkoxy oder Amino in Betracht kommen.

Reste R¹, R², R³, R⁴ und R⁵ sind z.B. Methyl, Ethyl, Propyl, Isopropyl, Butyl, Isobutyl, sec-Butyl, Cyclopropyl, Cyclobutyl, Cyclopentyl, Cyclohexyl oder Cycloheptyl.

Reste R¹, R², R³ und R⁴ sind weiterhin z.B. Fluor, Chlor oder Brom.

Reste R⁵ sind weiterhin z.B. Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, 2-Hydroxyethyl, 2-Hydroxypropyl, 3-Hydroxypropyl, 2-Hydroxybutyl, 4-Hydroxybutyl, 5-Hydroxypentyl, 6-Hydroxyhexyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 2-Fluorethyl, 2-Chlorethyl, 2-Bromethyl, 2- oder 3-Fluorpropyl, 2- oder 3-Chlorpropyl, 2- oder 3-Brompropyl, 2- oder 4-Fluorbutyl, 2- oder 4-Chlorbutyl, 2- oder 4-Brombutyl, 5-Fluorpentyl, 5-Chlorpentyl, 5-Brompentyl, 6-Fluorhexyl, 6-Chlorhexyl, 6-Bromhexyl, 2-Aminoethyl, 2- oder 3-Aminopropyl, 2- oder 4-Aminobutyl, 5-Aminopentyl oder 6-Aminohexyl.

Bevorzugt ist eine Verfahrensweise zur Herstellung von 2-Aminobenzaldehyden der Formel I, in der R¹, R², R³ und R⁴ jeweils Wasserstoff oder ein oder zwei Reste aus der Menge R¹, R², R³ und R⁴ Halogen oder C₁-C₄-Alkyl und die übrigen jeweils Wasserstoff bedeuten und der Ring A nicht benzoanelliert ist.

Bevorzugt ist weiterhin eine Verfahrensweise, in der man von Triazanonanen der Formel II ausgeht, in der R⁵ C₁-C₄-Alkyl oder C₂-C₄-Hydroxyalkyl bedeutet.

Besonders bevorzugt ist eine Verfahrensweise zur Herstellung von unsubstituiertem 2-Aminobenzaldehyd, d.h. die Reste R¹, R², R³ und R⁴ in Formel I bedeuten jeweils Wasserstoff und der Ring A ist nicht benzoanelliert.

Besonders bevorzugt ist weiterhin eine Verfahrensweise, in der man von Triazanonanen der Formel II ausgeht, in der R⁵ C₂-C₄-Hydroxyalkyl, insbesondere 2-Hydroxyethyl bedeutet.

Geeignete Säuren, die im erfindungsgemäßen Verfahren zur Anwendung gelangen können, sind in der Regel starke bis mittelstarke anorganische oder organische Säuren. Dabei sind insbesondere solche Säuren von Bedeutung, die mit Wasserdampf nicht flüchtig sind und weder beim Edukt noch beim Produkt Nebenreaktionen, z.B. Oxidationsreaktionen, verursachen. Beispielhaft seien folgende Säuren (oder deren saure Salze) genannt: Schwefelsäure, Natrium- oder Kaliumhydrogensulfat, Phosphorsäure, Natrium- oder Kaliumdihydrogenphosphat, Perchlorsäure, Benzolsulfonsäure oder p-Toluolsulfonsäure.

Die Verwendung von Schwefelsäure oder p-Toluolsulfonsäure ist bevorzugt, wobei p-Toluolsulfonsäure besondere Bedeutung zukommt.

In der Regel genügen katalytische Mengen an Säure zur Behandlung der Triazanonane II. Größere Mengen an Säure bis hin zu äquimolaren Mengen oder darüber hinaus können ebenfalls verwendet werden, bringen jedoch keinen technischen Vorteil.

Die Behandlung mit Säure erfolgt erfindungsgemäß in Gegenwart von Wasser, dabei verwendet man im allgemeinen 0,5 bis 5 l, vorzugsweise 1 bis 3 l und insbesondere ca. 2 l Wasser, jeweils bezogen auf 1 mol Triazanonan II.

Das erfindungsgemäße Verfahren, das sowohl in kontinuierlicher als auch diskontinuierlicher Arbeitsweise vorgenommen werden kann, wird zweckmäßig so durchgeführt, daß man Triazanonan II, Säure und Wasser in einer geeigneten Apparatur vorlegt und die Umsetzung bei einer Temperatur von 10 bis 120°C, vorzugsweise 70 bis 100°C und insbesondere 95 bis 100°C vornimmt. Geeignete Apparaturen zur Durchführung des neuen Verfahrens sind dabei übliche Vorrichtungen zur Durchführung einer Wasserdampfdestillation.

Wenn die obengenannte Temperatur erreicht ist, was in der Regel 15 bis 30 Minuten in Anspruch nimmt, wird mit der Einleitung von Wasserdampf begonnen.

Der Wasserdampf weist dabei in der Regel eine Temperatur von 102 bis 130°C, vorzugsweise 105 bis 115°C auf. Der Dampfdruck beträgt im allgemeinen 1 bis 3 bar, vorzugsweise 1 bis 1,5 bar.

Pro Liter vorgelegtem Reaktionsgemisch (Triazanonan II, Wasser und Säure) werden üblicherweise pro Stunde 0,1 bis 1,5 l, vorzugsweise 0,3 bis 0,8 l und insbesondere ca. 0,5 l Wasserdampf eingeleitet.

Die im erfindungsgemäßen Verfahren als Zielprodukte resultierenden 2-Aminobenzaldehyde I sind im allgemeinen wasserunlöslich, jedoch mit Wasserdampf leicht flüchtig, daher können sie in einer Vorlage aufgefangen werden, wobei sie dann im Kondensat als Niederschlag ausfallen. Zur Vervollständigung der Fällung können die 2-Aminobenzaldehyde unter Zusatz von Salzen, insbesondere von Natriumchlorid, ausgesalzen werden.

Auch eine Kühlung des Kondensats bewirkt eine beschleunigte Fällung, ohne daß dabei ein mikrokristallines Produkt erhalten wird. Die Abtrennung der 2-Aminobenzaldehyde aus dem Kondensat erfolgt auf an sich übliche Weise, z.B. durch Filtration oder Saugfiltration.

Mittels des erfindungsgemäßen Verfahrens fallen die 2-Aminobenzaldehyde der Formel I auf einfache Weise und in hoher Ausbeute und Reinheit an.

Wie eingangs bereits ausgeführt, sind 2-Aminobenzaldehyde wichtige Zwischenprodukte für die Synthese von Farbstoffen oder Wirkstoffen.

Die folgenden Beispiele sollen die Erfindung näher erläutern.

### Beispiel 1

In einem 4 l Rührreaktor wurden 300 g Dibenzotriazabicyclononan der Formel 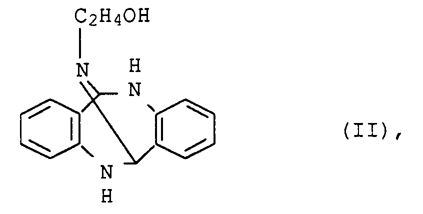
in 2 l Wasser vorgelegt, mit 10 g (0,105 mol) p-Toluolsulfonsäure versetzt und auf 100°C erhitzt. Anschließend wurden aus einem Dampferzeuger 5 l/h Wasserdampf mit einer Temperatur von 112°C und einem Druck von 1 bar in den Rührreaktor eingeleitet. Dabei wurden pro Stunde 1 Liter Kondensat aufgefangen, das auf 5°C gekühlt wurde. Die Wasserdampfdestillation wurde 6 Stunden lang durchgeführt. Es entstanden 180,8 g 2-Aminobenzaldehyd. Das entspricht einer Menge von 30,1 g pro Stunde. Eine Behandlung der Mutterlauge mit 300 g Natriumchlorid ergab weitere 2 g 2-Aminobenzaldehyd.

### Beispiel 2

In einem 4 l Rührreaktor wurden 150 g Dibenzotriazabicyclononan der Formel 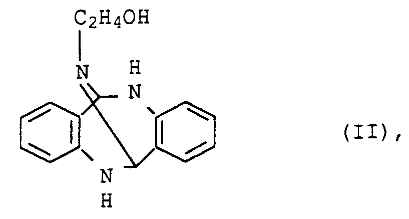
in 1 l Wasser vorgelegt, mit 5 ml konzentrierter Schwefelsäure versetzt und auf 100°C erhitzt. Anschließend wurden aus einem Dampferzeuger 7 l/h Wasserdampf mit einer Temperatur von 115°C und einem Druck von 1,2 bar in den Rührreaktor eingeleitet. Dabei wurden pro Stunde 1 Liter Kondensat aufgefangen, das auf 5°C gekühlt wurde. Die Wasserdampfdestillation wurde 2 Stunden lang durchgeführt. Es entstanden 52 g 2-Aminobenzaldehyd. Das entspricht einer Menge von 26 g pro Stunde.

## Patentansprüche

1. Verfahren zur Herstellung von 2-Aminobenzaldehyden der Formel I 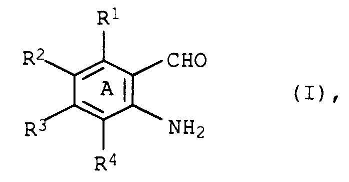 in der R¹, R², R³ und R⁴ gleich oder verschieden sind und unabhängig voneinander jeweils Wasserstoff, Halogen, C₁-C₄-Alkyl, C₃-C₇-Cycloalkyl oder Phenyl bedeuten und der Ring A benzoanelliert sein kann, durch Umsetzung von Triazanonanen der Formel II 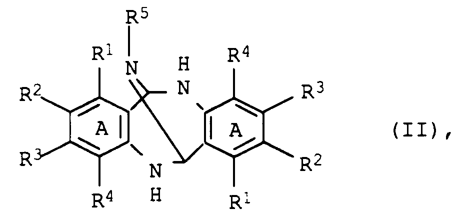 in der R¹, R², R³, R⁴ und der Ring A jeweils die obengenannte Bedeutung besitzen und R⁵ Wasserstoff, gegebenenfalls substituiertes C₁-C₁₀-Alkyl oder C₃-C₇-Cycloalkyl bedeutet, mit Säure, dadurch gekennzeichnet, daß man ein Triazanonan der Formel II bei einer Temperatur von 10 bis 120°C in Gegenwart von Wasser mit einer Säure behandelt und den resultierenden 2-Aminobenzaldehyd der Formel I mittels Wasserdampfdestillation abtrennt.

2. Verfahren nach Anspruch 1, dadurch gekennzeichnet, daß in Formel I und II R¹, R², R³ und R⁴ jeweils Wasserstoff oder ein oder zwei Reste aus der Menge R¹, R², R³ und R⁴ Halogen oder C₁₋₄-Alkyl und die übrigen jeweils Wasserstoff bedeuten und der Ring A nicht benzoanelliert ist.

3. Verfahren nach Anspruch 1, dadurch gekennzeichnet, daß in Formel II R⁵ C₁-C₄-Alkyl oder C₂-C₄-Hydroxyalkyl bedeutet.

4. Verfahren nach Anspruch 1, dadurch gekennzeichnet, daß man Schwefelsäure oder p-Toluolsulfonsäure als Säure verwendet.

## Claims

1. A process for preparing 2-aminobenzaldehydes of the formula I 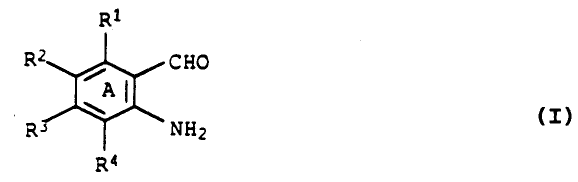 where R¹, R², R³ and R⁴ are identical or different and each is independently of the others hydrogen, halogen, C₁-C₄-alkyl, C₃-C₇-cycloalkyl or phenyl, and the ring A may be benzofused, by reacting triazanonanes of the formula II 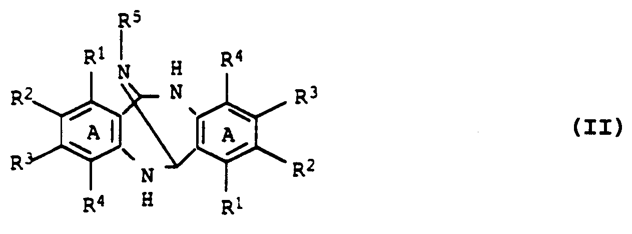 where R¹, R², R³, R⁴ and the ring A are each as defined above and R⁵ is hydrogen, substituted or unsubstituted C₁-C₁₀-alkyl or C₃-C₇-cycloalkyl, with an acid, which comprises treating a triazanonane of the formula II at from 10 to 120°C in the presence of water with an acid and separating off the resulting 2-aminobenzaldehyde of the formula I by steam distillation.

2. A process as claimed in claim 1, wherein, in the formulae I and II, R¹, R², R³ and R⁴ are each hydrogen or one or two radicals from R¹, R², R³ and R⁴ are halogen or C₁-C₄-alkyl and the others are each hydrogen and the ring A is not benzofused.

3. A process as claimed in claim 1, wherein, in formula II, R⁵ is C₁-C₄-alkyl or C₂-C₄-hydroxyalkyl.

4. A process as claimed in claim 1, wherein the acid used is sulfuric acid or p-toluenesulfonic acid.

## Revendications

1. Procédé de préparation de 2-aminobenzaldéhydes de formule I 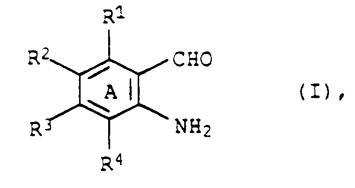 dans laquelle R¹, R², R³ et R⁴ sont identiques ou différents et représentent chacun, indépendamment les uns des autres, un atome d'hydrogène, d'halogène, un groupement alkyle en C₁-C₄, cycloalkyle en C₃-C₇ ou phényle et le noyau A peut être benzocondensé, par réaction de triazanonanes de formule II 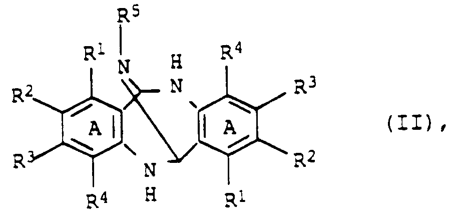 dans laquelle R¹, R², R³, R⁴ et le noyau A ont chacun la signification donnée ci-dessus et R⁵ représente un atome d'hydrogène, un groupement alkyle en C₁-C₁₀ ou cycloalkyle en C₃-C₇ éventuellement substitué, avec un acide, caractérisé en ce que l'on traite un triazanonane de formule II par un acide à une température de 10 à 120°C en présence d'eau, et on sépare le 2-aminobenzaldéhyde de formule I résultant par entraînement à la vapeur.

2. Procédé selon la revendication 1, caractérisé en ce que, dans les formules I et II, R¹, R², R³ et R⁴ représentent chacun un atome d'hydrogène ou bien l'un ou deux des restes R¹, R², R³ et R⁴ représentent des atomes d'halogène ou des restes alkyle en C₁-C₄ et les autres représentent chacun un atome d'hydrogène, et le noyau A n'est pas benzocondensé.

3. Procédé selon la revendication 1, caractérisé en ce que, dans la formule II, R⁵ représente un reste alkyle en C₁-C₄ ou hydroxyalkyle en C₂-C₄.

4. Procédé selon la revendication 1, caractérisé en ce que l'on utilise, comme acide, de l'acide sulfurique ou de l'acide p-toluènesulfonique.
